# EUROPEAN PATENT APPLICATION

(11) **EP 4 621 826 A1**
(43) Date of publication of application: **24.09.2025**
(21) Application number: 24164381.6
(22) Date of filing: 19.03.2024
(51) Int. Cl.: H01J 49/00, C07B 57/00, G01N 33/68, G01N 21/33, G01N 21/3577

(54) **METHOD FOR DETECTING NON-RACEMIC MIXTURES USING COLD ION PHOTOFRAGMENTATION MASS SPECTROMETRY**

(71) Applicant: ECOLE POLYTECHNIQUE FEDERALE DE LAUSANNE (EPFL), 1015 Lausanne (CH)
(72) Inventor: BOYARKINE, Oleg, 1121 Bremblens (CH); KOPYSOV, Vladimir, 1264 Saint-Cergue (CH)
(74) Representative: E. Blum & Co. AG

(57) **Abstract**

The present invention relates to an optical spectroscopic method for determining in absence of enantiopure standards of a target molecule whether a mixture of enantiomers of said molecule in a test composition is non-racemic. The method relies upon the combination of cold ion spectroscopy and mass spectroscopy detection. The method includes formation of diastereoisomers of the target molecule with each of the L- and D- enantiomers of a chiral reporter molecule and comparison of the photofragmentation spectra recorded for said diastereoisomers.

## Description

### FIELD OF THE INVENTION

The present invention relates to the technical field of optical spectroscopic methods for detecting non-racemic mixtures of enantiomers in a test composition.

### BACKGROUND OF THE INVENTION

Chirality plays a key role in biology as most of biologically relevant molecules are chiral, that is, non-superposable on their mirror images. Enantiomers are an isomeric pair of such molecules related to each other by a reflection, and by themselves, they therefore have identical physicochemical properties. However, enantiomers differ in how they interact with other chiral molecules. Moreover, living organisms demonstrate the dominance of one enantiomeric form over another, a phenomenon called homochirality. For instance, amino acids predominantly appear in the left-handed form, while monosaccharides - in the right-handed form. Due to the intrinsically chiral environment, enantiomers typically have substantially different biological properties, which makes chiral analysis essential for life sciences and drug design.

Identification of enantiomers, however, remains among the challenging tasks in analytical chemistry. Direct methods for chiral analysis are based on the difference in the interaction of a chiral molecule with left- and right-handed circularly polarized light and include techniques, such as electronic and vibrational circular dichroism (CD) and Raman optical activity (ROA) spectroscopy.

Alternatively, enantiomers can be differentiated by their interaction with other chiral molecules. For instance, nuclear magnetic resonance (NMR) spectroscopy, which is the "gold standard" for structural determinations, employs chiral derivatizing agents, which chemically react with enantiomers, or chiral solvating or lanthanide shift agents, which form non-covalent complexes with enantiomers. Such pairwise produced diastereomers then can be readily discriminated by their NMR spectra. The application of these techniques however is often limited by their relatively low sensitivity. In contrast, mass spectrometry (MS) is known for an exceptional sensitivity of analysis. MS measures the mass-to-charge ratio (m/z) of molecular ions and their fragments, which makes it virtually blind to enantiomers. Chiral analysis by MS, therefore is to precede by a separation technique, such as, for instance, chiral liquid chromatography (LC). In chiral LC, the resolution of enantiomers is typically achieved by the use of either chiral stationary phases (CSP) or chiral mobile phase additives (CMPA). Although chiral LC is considered as the method of choice for enantiomeric separation, it often requires choosing an appropriate column and a careful optimization of chromatographic conditions. Another separation technique widely used to resolve isomers prior to MS is ion mobility spectrometry (IMS), which separates gas-phase ions according to their mobility, when they travel through a buffer gas in an electric field. While IMS has demonstrated its ability to separate various types of isomers, enantiomers turned out to be a difficult case.

Cold ion spectroscopy (CIS), which is based on the measurement of ultraviolet (UV) or infrared (IR) photofragmentation spectra of cold gas-phase ions, has recently emerged as a technique capable of identifying isomers (Int. Rev. Phys. Chem. 2018, 37 (3-4), 559-606) . Cooling ions to cryogenic temperatures drastically suppresses the inhomogeneous spectral broadening, often allowing for vibrational resolution in UV and IR spectra, which makes them highly specific to the 3D structure of the ions. A library of such spectra recorded for a set of isomers can be used to identify them in their solution mixtures. This approach has been successfully employed for identification of isomeric peptides, oligosaccharides, lipids, and drug molecules. Enantiomers have identical UV and IR spectra. To identify and quantify each of the two enantiomers, Boyarkin et al (Anal. Chem. 2020, 92 (21), 14624-14632) proposed using a chiral UV chromophore to form non-covalent complexes with the enantiomers, record the spectra of the diastereomeric complexes and compare them with photofragmentation reference spectra of the diastereoisomeric complexes stored in a library. For recording the photofragmentation reference spectra, the enantiomerically pure standards are required. Enantiomerically pure standards are not available for all chiral molecules (e.g. a chemically not yet identified metabolite) or are obtainable by lengthy and cost-intensive purification procedures. Thus, this method cannot be applied for detecting non-racemic mixtures when the enantiomerically pure standards of the chiral molecule are not available.

In consequence, there is still a need for methods for determining in absence of the enantiomerically pure standards of a molecule whether a mixture of enantiomers of said substance is non-racemic .

### SHORT DESCRIPTION OF THE FIGURES

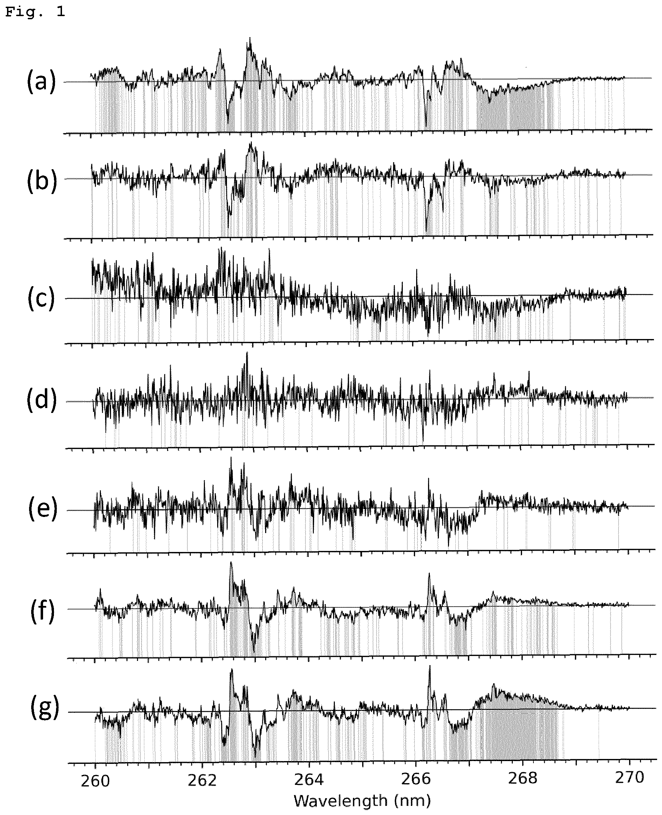

**Figure 1** shows the difference between UV photofragmentation spectra (photogragmentation yield versus light wavelength) averaged over multiple measurements of pairs of mixtures of L- and D-erythro-sphingosines either with L-Phe-L-Phe, or with D-Phe-D-Phe as chiral reporter molecule. The relative concentrations of L- and D-erythro-sphingosines shown in the figure are the same for each pair. The relative concentration of L-erythro-sphingosine decreases from the top panel (100%) to the bottom panel (0%): **a.** 100%; **b.** 80%; **c.** 60%; **d.** 50%; **e.** 40%; **f.** 20%; and **g.** 0%. The calculated p-values are: **a.** p<10⁻⁴, **b.** p<10⁻⁴, **c.** p<10⁻⁴, **d.** p=0.07, **e.** p=1.6·10⁻⁴, **f.** p< 10⁻⁴, **g.** p<10⁻⁴. The grey lines highlight the wavelengths at which the two photofragmentation spectra were assessed as being different at a significance level (a) of 5% according to a Welch's t-test. As attested by Figures **1****.b**, **1.c**, **1.e** and **1.f**, the grey area between the two spectra increases with the increase of the enantiomeric excess. As shown by Fig **1****.d**, within the accuracy of the experiment, the spectra of the 50%:50% mixture of L- and D-erythro-sphingosines with either with L-Phe-L-Phe or with D-Phe-D-Phe are identical (p >0.05). The indicated p-values are the results of a Fisher's combined probability test used for comparing the two spectra as a whole.

### SUMMARY OF THE INVENTION

Accordingly, it is an object of the present invention to provide an optical spectroscopic method for determining whether a mixture of enantiomers of a target molecule present in a test composition is non-racemic. The method comprises the following steps:
a) providing a test composition containing the mixture of enantiomers of said target molecule;
b) preparing diastereoisomers of the target molecule with an L-enantiomer of a chiral reporter molecule by combining a sample of the test composition with the L-enantiomer of the chiral reporter molecule;
c) preparing diasteroisomers of the target molecule with a D-enantiomer of the chiral reporter molecule by combining a further sample of the test composition with the D-enantiomer of the chiral reporter molecule;
d) ionization of the diastereoisomers to generate ions of the diasteroisomers;
e) recording by cold ion spectroscopy a photofragmentation spectrum for ions of the diastereoisomers of the target molecule with the L- enantiomer of the chiral reporter molecule, and for ions of the diastereoisomers of the target molecule with the D-enantiomer of the chiral reporter molecule;
f) determining whether the mixture of enantiomers of the target molecule is non-racemic based on a comparison of the photofragmetation spectrum recorded for the ions of the diastereoisomers of the target molecule with the L- enantiomer of the chiral reporter molecule with the photofragmetation spectrum recorded for the ions of the diastereoisomers of the target molecule with the D- enantiomer of the chiral reporter molecule.

### DETIALED DESCRIPTION OF THE INVENTION

Thus, it is an object of the present invention to address the need for a method for determining in absence of the enantiomerically pure standards of a molecule whether a mixture of enantiomers of said molecule in a test composition is non-racemic. The object is achieved by the optical spectroscopic method according to claim 1. Preferred embodiments are disclosed in the specification and the dependent claims.

The present invention will be described in more detail below.

Where the present description refers to "preferred" embodiments/features, combinations of these "preferred" embodiments/features are also deemed to be disclosed as long as the specific combination of the "preferred" embodiments/features is technically meaningful.

Unless otherwise stated, the following definitions shall apply in this specification:
As used herein, the term "a", "an", "the" and similar terms used in the context of the present invention (especially in the context of the claims) are to be construed to cover both the singular and plural unless otherwise indicated herein or clearly contradicted by the context.

As used herein, the term "and/or" means that either all or only one of the elements of said group may be present. For example, "A and/or B" means "only A, or only B, or both A and B". In the case of "only A", the term also covers the possibility that B is absent, i.e. "only A, but not B".

As used herein, the terms "including", "containing" and "comprising" are used herein in their open-ended, non-limiting sense. It is understood that the various embodiments, preferences and ranges may be combined at will. Thus, for instance a solution comprising a compound A may include other compounds besides A. However, the term "comprising" also covers, as a particular embodiment thereof, the more restrictive meanings of "consisting essentially of" and "consisting of, so that for instance "a solution comprising A, B and optionally C" may also (essentially) consist of A and B, or (essentially) consist of A, B and C. As used herein, the transitional phrase "consisting essentially of" (and grammatical variants) is to be interpreted as encompassing the recited materials or steps and those that do not materially affect the basic and novel characteristic (s) of the claimed invention. Thus, the term "consisting essentially of" should not be interpreted as equivalent of "comprising".

As used herein, the term "about" means that the amount or value in question may be the specific value designated or some other value in its neighborhood. Generally, the term "about" denoting a certain value is intended to denote a range within ± 5 % of the value. As one example, the phrase "about 100" denotes a range of 100 ± 5, i.e. the range from 95 to 105. Preferably, the range denoted by the term "about" denotes a range within ± 3 % of the value, more preferably ± 1 %. Generally, when the term "about" is used, it can be expected that similar results or effects according to the invention can be obtained within a range of ±5 % of the indicated value.

Surprisingly, it has been found that an optical spectroscopic method comprising the following steps:
a) providing a test composition containing the mixture of enantiomers of said target molecule;
b) preparing diastereoisomers of the target molecule with an L-enantiomer of a chiral reporter molecule by combining a sample of the test composition with the L-enantiomer of the chiral reporter molecule;
c) preparing diasteroisomers of the target molecule with a D-enantiomer of the chiral reporter molecule by combining a further sample of the test composition with the D-enantiomer of the chiral reporter molecule;
d) ionization of the diastereoisomers to generate ions of the diasteroisomers;
e) recording by cold ion spectroscopy a photofragmentation spectrum for ions of the diastereoisomers of the target molecule with the L- enantiomer of the chiral reporter molecule, and for ions of the diastereoisomers of the target molecule with the D-enantiomer of the chiral reporter molecule;
f) determining whether the mixture of enantiomers of the target molecule is non-racemic based on a comparison of the photofragmetation spectrum recorded for ions of the diastereoisomers of the target molecule with the L- enantiomer of the chiral reporter molecule with the photofragmetation spectrum recorded for the ions of the diastereoisomers of the target molecule with the D- enantiomer of the chiral reporter molecule; allows to determine whether the mixture of enantiomers of the target molecule contained in the test composition is non-racemic. Advantageously, the optical spectroscopic method according to the present invention is based on the comparison of the photofragmentation spectrum recorded for the ions of diastereoisomers of the target molecule with the L- enantiomer of the chiral reporter molecule with the photofragmetation spectrum recorded for the ions of the diastereoisomers of the target molecule with the D- enantiomer of the chiral reporter molecule. Hence, the present method does not require the use of a library of photofragmentation reference spectra, which can be generated only with enantiopure standards of the target molecule, and is particularly useful for detecting non-racemic mixtures for which the enantiopure standards are not available. Further, the present method relies upon the difference between the electric transition dipole moments and consequently, is significantly more sensitive (few orders of magnitude) than the generally employed circular dichroism (CD) spectroscopy, which relies upon the difference between the magnetic dipole moments. Consequently, a significantly lower amount of mixture of enantiomers is required in the present method compared to CD spectroscopy. Additionally, the present method includes mass spectroscopy detection and enables selection of the target molecule in a test composition based on its molecular weight, which is not achievable with CD spectroscopy.

As used herein, the term "target molecule" refers to a chiral molecule (i.e. a molecule or an ion that cannot be superimposed with its mirror image) characterized by a molecular weight, preferably from about 50 to about 2000 Daltons. The structure of the target molecule may be known or unknown. Preferably, the target molecule is a lipid, a carbohydrate, a peptide and a small molecule drug, more preferably a lipid, a peptide or a small molecule drug.

The term "mixture of enantiomers" refers to a mixture containing the D- and the L- enantiomer of the target molecule at unknown relative concentrations conc^{D} and conc^{L}, respectively with conc^{D} + conc^{L} = 1. A mixture of enantiomers is non-racemic when the relative concentrations of the D- and the L- enantiomer of the target substance are different i.e. conc^{D} ≠ conc^{L}.

The test composition provided at step a) may be in a liquid or in a solid form. In one embodiment, the test composition may consist of the mixture of enantiomers of the target substance. In another embodiment, the test composition may further contain
- isomers other than the enantiomers of the target molecule (e.g. diastereoisomers; structural isomers); and/or
- two or more mixtures of enantiomers of two or more target molecules, wherein each of said two or more target molecules has a different molecular weight; and/or
- an achiral substance having a molecular weight different from the molecular weight of the target molecule(s); and/or
- a solvent, such as water, an organic solvent, preferably a water-miscible solvent, and mixtures thereof.

At steps b) and c) of the method, diastereoisomers are prepared by combining a sample of the test composition (i.e. a sample containing a mixture of the L-enantiomer and of the D- enantiomer of the target molecule) with an L-enantiomer of the chiral reporter molecule, and a further sample of the test composition (i.e. a sample containing a mixture of the L-enantiomer and of the D-enantiomer of the target molecule) with a D-enantiomer of the chiral reporter molecule. Thus, at step b) for each target molecule, two diastereoisomers of said target molecule with the L-enantiomer of the chiral reporter molecule are formed, namely (L-target molecule)- (L-chiral reporter molecule) and (D-target molecule)- (L-chiral reporter molecule), while at step c) for each target molecule, two diastereoisomers of said target molecule with the D- enantiomer of the chiral reporter molecule are formed, namely (L-target molecule)- (D-chiral reporter molecule) and (D-target molecule)- (D-chiral reporter molecule). The amount of the sample used at step b) is identical to the amount of the further sample used at step c) so that the concentration of the mixture of enantiomers is the same in the two samples. Also, the procedures applied at steps b) and c) for combining the target molecule with the chiral reporter molecule are identical. Also preferably the molar ratio of the chiral reporter molecule to the enantiomers of the target molecule is at least 1, more preferably higher than 1.

As used herein, the term "chiral reporter molecule" refers to an optically active molecule that is available, preferably commercially available, both as an enantiomerically enriched D-enantiomer and as enantiomerically enriched L-enantiomer. Herein, enantiomerically enriched refers to a percent of enantiomeric excess (% ee) of at least 80%, preferably of at least 90%, more preferably of at least 95%, much preferably enantiopure. The chiral reporter molecule is preferably soluble in a solvent typically used in electrospray ionization analytical methods, such as water, methanol, acetonitrile, and mixtures thereof. Preferably, the chiral reporter molecule is prone to ionization, in particular to electrospray ionization. If the target molecule does not undergo ionization, then the chiral reporter molecule must be prone to ionization.

Depending on the structures of the target molecule and of the chiral reporter molecule and the applied conditions during steps b) and c), either non-covalent complexes, or chemical products are formed between the enantiomers of the target molecules and the L-enantiomer or the D-enantiomer of the chiral reporter molecule. Hence, the term "combining" includes both the physical mixture of the samples with the chiral reporter molecule followed by forming non-covalent bonds between them, and the physical mixture of the samples with the chiral reporter molecule followed by the chemical reaction between the enantiomers of the target molecule and the enantiomers of the chiral reporter molecule to form chemical products. If required, the samples of the test composition or the chiral reporter molecules may be diluted or dissolved in a solvent, preferably same solvent, prior to being combined. The solvent is preferably a solvent typically used in electrospray ionization analytical methods, such as water, methanol, acetonitrile, and mixtures thereof.

In one embodiment, the diastereoisomers of the target molecule with the chiral reporter molecule are non-covalent complexes formed between the target molecule and the chiral reporter molecule. As used herein, the term "non-covalent complex" refers to a complex formed between an enantiomer (either L- or D-) of the target enantiomer and an enantiomer (either L- or D-) of the chiral reporter molecule that results from a non-covalent interaction between the enantiomer (either L- or D-) of the target molecule and the enantiomer (either L- or D-) of the chiral reporter molecule. Thus, at step b) for each target molecule contained in the test composition as a mixture of enantiomers, two non-covalent diastereoisomeric complexes, namely (L-target molecule)- (L-chiral reporter molecule) and (D-target molecule)- (L-chiral reporter molecule) are formed. In a similar manner at step c) for each target molecule contained in the test composition as a mixture of enantiomers, two non-covalent diastereoisomeric complexes, namely (L-target molecule)- (D-chiral reporter molecule) and (D-target molecule)- (D-chiral reporter molecule) are formed.

In another embodiment, the diastereoisomers of the target molecule with the chiral reporter molecule are chemical products resulting from a chemical reaction of the target molecule with the chiral reporter molecule. As used herein, the term "chemical product" refers to the product resulting from a chemical reaction between an enantiomer (either L- or D-) of the target substance and an enantiomer (either L- or D-) of the chiral reporter molecule. Thus, if a chemical reaction occurs at step b) for each target molecule contained in the test composition as a mixture of enantiomers, two diastereoisomeric chemical products, namely (L-target molecule)- (L-chiral reporter molecule) and (D-target molecule)- (L-chiral reporter molecule) are formed. In a similar manner if a chemical reaction occurs at step c) for each target molecule contained in the test composition as a mixture of enantiomers, two diastereoisomeric chemical products, namely (L-target molecule)- (D-chiral reporter molecule) and (D-target molecule)- (D-chiral reporter molecule) are formed.

At steps c), the diastereoisomers prepared at step b) or c) are subjected to ionization to generate ions of said diastereoisomers. Preferably, the ionisation is electrospray ionization (ESI), more preferably nano-electrospray ionization (n-ESI) .

Subsequently, a photofragmentation spectrum (fragmentation yield (%) *versus* light wavelength) is recorded by cold ion spectroscopy for ions of the diastereoisomers of the target molecule with the L- enantiomer of the chiral reporter molecule (i.e. (L-target molecule)-(L-chiral reporter molecule); (D-target molecule)-(L-chiral reporter molecule)), and for ions of the diastereoisomers of the target molecule with the D-enantiomer of the chiral reporter molecule (i.e. (L-target molecule)-(D-chiral reporter molecule); (D-target molecule)-(D-chiral reporter molecule)). In one embodiment, the photogramentation spectrum is recorded for all ions generated at step d). In an alternative embodiment, the photogramentation spectrum is recorded for some of the ions generated at step d). In this specific embodiment, a quadrupole mass filter may be used for selecting based on a mass-to-charge ratio the ion(s) for which the photofragmentation spectrum is recorded. If the test composition contains two or more target molecules with each of said target substances being present as a mixture of enantiomers in the test composition, and each of said two or more target molecules having a different molecular weight, then for each of said target molecules a photofragmentation spectrum is recorded for ions of diastereoisomers of said target molecule with the L-enantiomer of the chiral reporter molecule and for ions of diastereoisomers of said molecule with the D-enantiomer of the chiral reporter molecule. In such case, for each target molecule, a selection of the ion(s) based on a mass-to-charge ratio with a quadrupole mass filter is required prior to the recording of the photofragmentation spectrum.

Steps d) and e) conducted for the diastereoisomers with the L-enantiomer of the chiral reporter molecule, may be conducted in parallel on different instruments or in a sequence on the same instrument with steps d) and e) for the diastereoisomers with the D-enantiomer of the chiral reporter molecule.

The recording of the photofragmentation spectrum by cold ion spectroscopy may comprise the following steps:
e-1) cryogenically cooling the ions of the diastereoisomers in an ion trap;
e-2) irradiating the ions of step e-1) with laser light at n wavelengths (λ_{1, ...} λₙ), n ≥ 1, to generate charged ion fragments thereof; and
e-3) measuring at each of the n wavelengths, for each of the charged ion fragments detected by mass spectrometry and generated both from the ions of the diasteroisomers with the L-enantiomer of the chiral reporter molecule and from the ions of diastereoisomers with the D-enantiomer of the chiral reporter molecule, a fragmentation yield ^{L}S for the diasteroisomers with the L-enantiomer of the chiral reporter molecule, and a fragmentation yield ^{D}S for the diasteroisomers with the D-enantiomer of the chiral reporter molecule, respectively.

At step e-1) at least one of the ions generated at step d) both from the diastereoisomers with the L-enantiomer of the chiral reporter molecule and the diastereoisomers with the D-enantiomer of the chiral reporter molecule is cryogenically cooled in an ion trap. Preferably, the at least one ion is cryogenically cooled to a temperature lower than 20 K, for instance to about 10K. For this purpose, the ion trap is configured to be cooled to a temperature below ambient temperature and contains a gas that is non-condensing at the trap temperature. This so-called cooling gas preferably is pulsed into the trap through a pulsed molecular valve. The gas is pulsed typically 0.1-1 ms before the arrival of the ions to the trap, and typically at least 5-20 ms prior to the first light pulse. The peak pressure of cooling gas in the ion trap is typically 0.5 mbar. The cooling gas is usually cooled through ion trap. Thereby, the ions may cool through collisions with the cooling gas. The cooling brings the ionic species in the ion trap to their respective ground vibrational quantum state as by Boltzmann distribution.

The cryogenically cooled ions are subsequently irradiated with laser light at n wavelengths (λ_{1, ...} λₙ), n ≥ 1, to generate charged ion fragments thereof. The irradiation preferably occurs while the ions are in the ion trap. For this purpose, one or two optical windows are provided in the apparatus such that the light may be directed through the one or two windows into the ion trap, preferably along the trap axis. In one embodiment, the irradiation is conducted at a single wavelength (n = 1) with, for instance, a wavelength non-tunable laser. In another embodiment, the irradiation is conducted with laser light at n different wavelengths, wherein n is higher than 10, preferably higher than 100, more preferably higher than 1000. Preferably, the n different wavelengths differ from each other by at least 0.05 nm. In such embodiment, a tunable laser may be used for irradiation. Herein, the term "laser" is intended to include devices that produce a laser light, including an optical parametric oscillator (OPO). In a preferred embodiment, the laser is an optical parametric oscillator (OPO).

At step e-3), at each of the n wavelengths, for each of the charged ion fragments detected by mass spectrometry and generated both from the ions of the diasteroisomers with the L-enantiomer of the chiral reporter molecule and from the ions of diastereoisomers with the D-enantiomer of the chiral reporter molecule, a fragmentation yield ^{L}S is measured for the diasteroisomers with the L-enantiomer of the chiral reporter molecule, and a fragmentation yield ^{D}S is measured for the diasteroisomers with the D-enantiomer of the chiral reporter molecule. As well known to the skilled person, the "fragmentation yield" of a charged ion fragment corresponds to the ratio of the number of charged ion fragments of a specific mass-to-charge ratio to the number of parent ions (i.e. the ions from which the charged ion fragments were generated by irradiation) irradiated.

The method further comprises step f), namely determining whether the mixture of enantiomers of the target molecule is non-racemic based on a comparison of the photofragmetation spectrum recorded for ions of diastereoisomers of the target molecule with the L- enantiomer of the chiral reporter molecule with the photofragmetation spectrum recorded for ions of the diastereoisomers of the target molecule with the D- enantiomer of the chiral reporter molecule. As the decision is made solely by comparison of the two spectra, there is no need of enantiomerically pure enantiomers of the target molecule as standards.

The photofragmentation spectrum recorded at step e) may be an UV photofragmentation spectrum i.e. the light used for obtaining said photofragmentation spectrum is an UV light. In such case, the target molecule and/or the chiral reporter molecule contains an UV chromophore, specifically an UV chromophore absorbing in the UV spectral range of the UV laser light used for radiation. In an embodiment, the target molecule does not contain an UV chromophore and the chiral reporter molecule contains an UV chromophore. Particularly useful chiral reporter molecules containing an UV chromophore include, but are not limited to natural or chemically modified aromatic amino acids (e.g. tryptophane, tyrosine, phenylalanine, histidine), dipeptides containing at least one residue of said natural or chemically modified aromatic amino acids (e.g. L-phenylalanine- L-phenylalanine; D-phenylalanine-D-phenylalanin), and tripeptides containing at least one residue of said natural or chemically modified aromatic amino acids.

The photofragmentation spectrum recorded at step e) may be an IR photogragmentation spectrum i.e. the light used for obtaining said photofragmentation spectrum is an IR light. In such case, the chiral reporter molecule is preferably a chiral solvent, more preferably a chiral alcohol, such as sec-butanol (2-butanol).

The method may further comprise a step g) conducted between step d) and e):
g) selecting one or more ions from the ions of the diastereosiomers based on a mass-to-charge ratio using a quadrupole mass filter. This step allows isolation of the ions of the diastereoisomers of the target molecule with the chiral reporter molecule from the remaining of the sample, and is particularly useful when the sample contains several target molecules.

It is further preferred that m1 photofragmentation spectra are recorded for ions of diastereoisoimers of the target molecule with the L-enantiomer of the chiral reporter molecule and/or m2 photofragmentation spectra are recorded for ions the diastereoisomers of the target molecule with the D-enantiomer of the chiral reporter molecules. The integers m1 and m2 are independently of each other ≥ 2. If the photofragmentation spectra are measured for a certain number of times (m1, m2), the method further comprises step h) conducted between steps e) and f)
h) calculating at each of the n wavelengths, for each of the one or more charged ion fragments detected by mass spectrometry and generated both from the ions of the diasteroisomers with the L-enantiomer of the chiral reporter molecule and from the ions of diastereoisomers with the D-enantiomer of the chiral reporter molecule, an average fragmentation yield <^{L}S> at said wavelength for the diasteroisomers with the L-enantiomer of the chiral reporter molecule and/or an average ion signal <^{D}S> at said wavelength for the diasteroisomers with the D-enantiomer of the chiral reporter molecule.

If the photofragmentation spectra are measured for a certain number of times (m1, m2), the method preferably further comprises step i) conducted between step e) and step f):
i) calculating at each of the n wavelengths, for each of the one or more charged ion fragments detected by mass spectrometry and generated both from the ions of the diasteroisomers with the L-enantiomer of the chiral reporter molecule and from the ions of diastereoisomers with the D-enantiomer of the chiral reporter molecule, a statistical error δ^{L}S for the fragmentation yield at said wavelength for the diasteroisomers with the L-enantiomer of the chiral reporter molecule and/or a statistical error δ^{L}S for the fragmentation yield at said wavelength for the diasteroisomers with the D-enantiomer of the chiral reporter molecule.

Preferably, the integers m1 and m2 are independently of each other ≥ 2. In such case, the method preferably contains as step j) conducted between steps h) and f)
j) calculating at each of the n wavelengths, for each of the one or more charged ion fragments detected by mass spectrometry and generated both from the ions of the diasteroisomers with the L-enantiomer of the chiral reporter molecule and from the ions of diastereoisomers with the D-enantiomer of the chiral reporter molecule, a difference in absolute value ΔS at said wavelength between the average fragmentation yield at said wavelength for the diasteroisomers with the L-enantiomer of the chiral reporter molecule <^{L}S> and the average fragmentation yield for the diasteroisomers with the D-enantiomer of the chiral reporter molecule at said wavelength <^{D}S>. The term "difference in absolute value" corresponds to an absolute value of the difference between <^{L}S> and <^{D}S>.

If the integers m1 and m2 are independently of each other ≥ 2, then the method may further contain step k) conducted between steps i) and f)
k) calculating at each of the n wavelengths, for each of the one or more charged ion fragments detected by mass spectrometry and generated both from the ions of the diasteroisomers with the L-enantiomer of the chiral reporter molecule and from the ions of diastereoisomers with the D-enantiomer of the chiral reporter molecule, an average statistical error ΔδS for the fragmentation yield at said wavelength based on the statistical error δ^{L}S for the fragmentation yield at said wavelength for the diasteroisomers with the L-enantiomer of the chiral reporter molecule and the statistical error δ^{L}S for the fragmentation yield at said wavelength for the diasteroisomers with the D-enantiomer of the chiral reporter molecule.

In the present method, the comparison may include comparing for each of the one or more charged ion fragments detected by mass spectrometry and generated both from the ions of the diasteroisomers with the L-enantiomer of the chiral reporter molecule and from the ions of diastereoisomers with the D-enantiomer of the chiral reporter molecule and at each of the n wavelengths, the absolute difference ΔS with a predetermined statistical error.

Alternatively, the comparison may include comparing for each of the one or more charged ion fragments detected by mass spectrometry and generated both from the ions of the diasteroisomers with the L-enantiomer of the chiral reporter molecule and from the ions of diastereoisomers with the D-enantiomer of the chiral reporter molecule and at each of the n wavelengths, the absolute difference ΔS with either the statistical error δ^{L}S for the fragmentation yield for the diasteroisomers with the L-enantiomer of the chiral reporter molecule, or the statistical error δ^{D}S for the fragmentation yield for the diasteroisomers with the D-enantiomer of the chiral reporter molecule.

In a further alternative, the comparison at step f) may include comparing for each of the one or more charged ion fragments detected by mass spectrometry and generated both from the ions of the diasteroisomers with the L-enantiomer of the chiral reporter molecule and from the ions of diastereoisomers with the D-enantiomer of the chiral reporter molecule and at each of the n wavelengths, the absolute difference ΔS with the average statistical error ΔδS for the fragmentation yield at said wavelength.

The method may be implemented on an instrument including:
- a device for ionization and gasification of the diastereoisomers, preferentially using electrospray ionization technique;
- optionally a mass filter that allows for selection of the ionized target molecules or of the diastereoisomers;
- a cryogenic ion trap that allows for trapping and cooling of the preselected ions and, in the case of selecting the ionized target molecules, for injection of chiral reporter molecules into it for the subsequent formation of diastereoisomers;
- a tunable IR or visible or UV source of laser light to perform action dissociation vibrational or electronic spectroscopy or both;
- a device for mass-selective detection of the number of the charged ion fragments resulting from laser action on the diastereoisomers or for detecting the change in the number of the ions of diastereoisomers after irradiation with light.

### EXAMPLES

To further illustrate the invention, the following **examples** are provided. These examples are provided with no intend to limit the scope of the invention.

### Materials and Methods

### a) Chemicals

L- and D-erythro-sphingosines (ES) (≥98% purity) were purchased from Cayman Chemical; L-Phe-L-Phe and D-Phe-D-Phe (≥98% purity) were purchased from Bachem.

Water and acetic acid (Optima^{™}, LC-MS grade) were purchased from Fisher Scientific; isopropyl alcohol (CHROMASOLV^{™}, LC-MS grade) was purchased from Honeywell.

Individual stock solution of amino acids and dipeptides and of *erythro*-sphingosines (ES) were prepared in water and isopropyl alcohol, respectively. All the solutions for UV spectroscopy measurements had the following composition: 50 µM of an amino acid or a dipeptide and 50 µM of ESs in a mixture of water and isopropyl alcohol (50:50) with 1% of acetic acid.

### b) Experimental method

The ions, produced in the gas phase from solution by a nano-electrospray ionization (n-ESI) source, enter to a triple skimmer differentially pumped chamber through a 100 mm long stainless-steel capillary of 0.7 mm ID. The ions are then accumulated and thermalized in an octupole ion trap for 97 ms, before they are released and pass through a quadrupole mass filter (Q1), which is set to select the ions of interest. Mass-selected ions are then turned by 90° using an electrostatic bender, focused by a stack of three electrostatic lenses and moved through an RF octupole guide into a cold octupole trap, which is kept at 6 K. The trap is driven by two 1 MHz sinus waveforms with peak-to-peak amplitudes of 50-100 V. The stored in the trap non-covalent complexes are cooled down to ~10 K in collisions with He buffer gas, which is pulsed into the trap shortly before arrival of the ions. Approximately 85 ms later the ions are irradiated by a pulse of UV light (1.8±0.7 mJ/pulse, 5 ns duration, ~6 cm⁻¹ spectral linewidth), produced by a UV optical parametric oscillator (OPO) and undergo photo fragmentation. The fragment and parent ions were 90° turned by the second electrostatic quadrupole bender either toward a highly sensitive quadrupole mass spectrometer (QMS) or toward high-resolution broadband Orbitrap-based MS (Exactive, Thermo Fisher). UVPF mass spectra are measured by continuously recording the intensity of the photofragments and the remaining parent ions using the Orbitrap-based MS while scanning UV wavelength. The repetition rate of the cooling/fragmentation cycle was determined by the 10 Hz repetition rate of the OPO, such that the parent ions experienced only one OPO shot in each cycle. At each UV wavelength the whole mass spectrum of the fragments and the remaining parent ions was measured in 10 cycles and averaged to give a fragment MS in 2D UV-MS fingerprint. The measured ion signals were normalized to OPO pulse energy, which was measured by a broadband pyroelectric detector. The whole fragment mass spectrum was normalized to the total ion signal detected in the cycle.

**Data processing.** The measured 2D UV-MS data arrays (intensity vs UV wavelength and m/z) were, first, pre-processed for detection of mass-peaks using an in-house software package. To determine the overall fragmentation yield, resulting reduced 2D data array was integrated over m/z coordinate for all the fragment peaks, except the parent ion. Alternatively, the data array can be integrated over a few (or a single) fragment mass peaks to determine the yield for these fragments. In both cases, the resulting 1D array represents a UV photofragmentation spectrum (dependence of fragmentation yield on UV wavelength).

The difference between the UV photofragmentation spectra recorded for mixtures of L/D enantiomers of *erythro*-sphingosine (ES) in complexes with L-Phe-L-Phe and D-Phe-D-Phe reporter molecule is depicted in **Fig. 1****.** The relative concentrations of L-ES and D-ES and the calculated p-values are: **a.** 100/0% and p<10⁻⁴, **b.** 80/20% and p<10⁻⁴, **c.** 60/40% and p<10⁻⁴, **d.** 50/50% and p=0.07, **e.** 40/60% and p=1.6·10⁻⁴, **f.** 20/80% and p< 10⁻⁴, **g.** 0/100% and p<10⁻⁴. The grey lines highlight the wavelengths at which the differences exceed the predetermined spectral noise at a significance level (a) of 5% according to a Welch's t-test (p < 0.05). As attested by Figures **1****.b**, **1.c**, **1.e** and **1.f**, the grey area between the two spectra increases with the increase of the enantiomeric excess. As shown by Fig **1****.d**, within the accuracy of the experiment, the spectra of the 50%:50% mixture of L- and D-erythro-sphingosines with either with L-Phe-L-Phe or with D-Phe-D-Phe are identical (p >0.05). The indicated p-values are the results of a Fisher's combined probability test used for comparing the two spectra as a whole.

## Claims

1. An optical spectroscopic method for determining whether a mixture of enantiomers of a target molecule is non-racemic, comprising the following steps:
a) providing a test composition containing the mixture of enantiomers of said target molecule;
b) preparing diastereoisomers of the target molecule with an L-enantiomer of a chiral reporter molecule by combining a sample of the test composition with the L-enantiomer of the chiral reporter molecule;
c) preparing diasteroisomers of the target molecule with a D-enantiomer of the chiral reporter molecule by combining a further sample of the test composition with the D-enantiomer of the chiral reporter molecule;
d) ionization of the diastereoisomers to generate ions of the diasteroisomers;
e) recording by cold ion spectroscopy a photofragmentation spectrum for ions of the diastereoisomers of the target molecule with the L- enantiomer of the chiral reporter molecule, and for ions of the diastereoisomers of the target molecule with the D-enantiomer of the chiral reporter molecule;
f) determining whether the mixture of enantiomers of the target molecule is non-racemic based on a comparison of the photofragmetation spectrum recorded for ions of diastereoisomers of the target molecule with the L- enantiomer of the chiral reporter molecule with the photofragmetation spectrum recorded for ions of the diastereoisomers of the target molecule with the D- enantiomer of the chiral reporter molecule.

2. The optical spectroscopic method according to claim 1, wherein the diastereoisomers are non-covalent complexes formed between the target molecule and the chiral reporter molecule.

3. The optical spectroscopic method according to claim 1, wherein the diastereoisomers are chemical products resulting from a chemical reaction of the target molecule with the chiral reporter molecule.

4. The optical spectroscopic method according to any one of the previous claims, wherein the test composition further contains isomers other than the enantiomers of the target substance.

5. The optical spectroscopic method according to any one of the previous claims, wherein the test composition contains two or more mixtures of enantiomers of two or more target molecules with each of said two or more target molecules having a different molecular weight.

6. The optical spectroscopic method according to any one of the previous claims, wherein the target substance is a lipid, a carbohydrate, a peptide, or a small molecule drug.

7. The optical spectroscopic method according to any one of claims 1 to 6, wherein the photogragmentation spectrum is an UV photofragmentation spectrum.

8. The optical spectroscopic method according to claim 7, wherein the target molecule and/or the chiral reporter molecule contains an UV chromophore.

9. The optical spectroscopic method according to claim 7, wherein the target molecule does not contain an UV chromophore and the chiral reporter molecule contains an UV chromophore.

10. The optical spectroscopic method according to any one of claims 1 to 6, wherein the photogragmentation spectrum is an IR photofragmentation spectrum.

11. The optical spectroscopic method according to claim 10, wherein the chiral reporter molecule is a chiral solvent, preferably sec-butanol.

12. The optical spectroscopic method according to any one of the previous claims, wherein step e) comprises
e-1) cryogenically cooling the ions of the diastereoisomers in an ion trap;
e-2) irradiating the ions of step e-1) with laser light at n wavelengths (λ_{1, ...} λₙ), n ≥ 1, to generate charged ion fragments thereof; and
e-3) measuring at each of the n wavelengths, for each of the charged ion fragments detected by mass spectrometry and generated both from the ions of the diasteroisomers with the L-enantiomer of the chiral reporter molecule and from the ions of diastereoisomers with the D-enantiomer of the chiral reporter molecule, a fragmentation yield ^{L}S for the diasteroisomers with the L-enantiomer of the chiral reporter molecule, and a fragmentation yield ^{D}S for the diasteroisomers with the D-enantiomer of the chiral reporter molecule, respectively.

13. The optical spectroscopic method according to claim 12, wherein n is 1.

14. The optical spectroscopic method according to any one of the previous steps, further comprising a step g) conducted between step d) and e)
g) selecting one or more ions from the ions of the diastereosiomers based on a mass-to-charge ratio using a quadrupole mass filter.

15. The optical spectroscopic method according to any one of the preceding claims, wherein m1 photofragmentation spectra are recorded for ions of diastereoisoimers of the target molecule with the L-enantiomer of the chiral reporter molecule and/or m2 photofragmentation spectra are recorded for ions the diastereoisomers of the target molecule with the D-enantiomer of the chiral reporter molecules, with m1 and m2 being independently of each other ≥ 2.
